# EUROPEAN PATENT SPECIFICATION

(11) **EP 0 768 083 B1**
(45) Date of publication and mention of the grant of the patent: **26.03.2003**
(21) Application number: 96304906.9
(22) Date of filing: 03.07.1996
(51) Int. Cl.: A61K 31/135

(54) **Sertraline for treating post myocardial infarction patients**
Sertralin zur Behandlung von Post-Myocard-Infarkt-Patienten
Sertraline pour le traitement des patients post-infarction myocardique

(30) Priority: 17.07.1995 US 1437
(43) Date of publication of application: 16.04.1997
(73) Proprietor: PFIZER INC., New York, N.Y. 10017 (US)
(72) Inventor: Johnson, Brian F., East Lyme, Connecticut 06333 (US)
(74) Representative: Moore, James William

(56) References cited:
- US-A- 5 114 976

## Description

This invention relates to the use of the compound (1S-cis)-4-(3,4-dichlorophenyl)-1,2,3,4-tetrahydro-N-methyl-1-naphthalenamine, hereinafter referred to by its generic name "sertraline", or a pharmaceutically acceptable salt thereof for the preparation of a medicament for treating post myocardial infarction patients.

Sertraline, which has the empiral formula C₁₇H₁₇NCl₂ and the structural formula 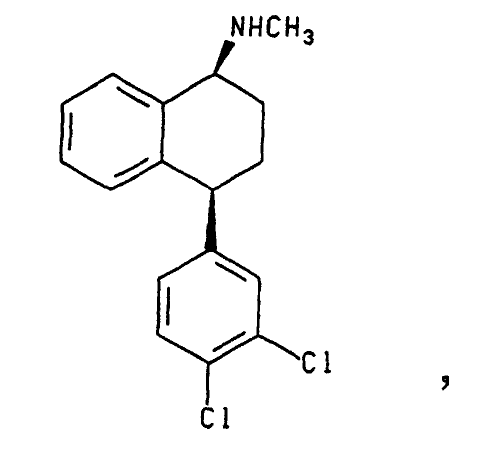 is a known antidepressant and anorectic agent. United States Patent 4,536,518, which issued on August 20, 1985, discloses sertraline and related compounds of the formula 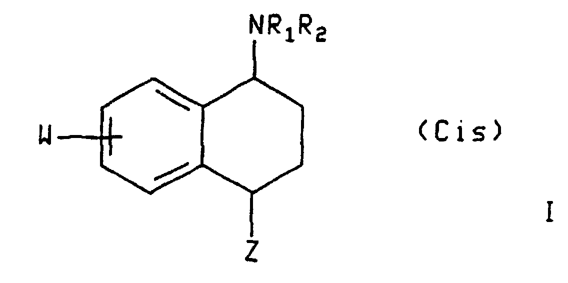 wherein Z is 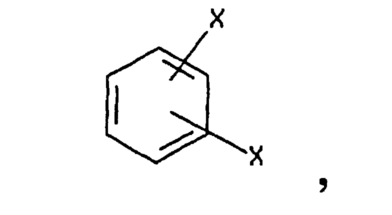 and R₁, R₂, W, X and Y are as defined therein, and states that such compounds exhibit antidepressant and anorectic activity in vivo in mammals.

United States Patent 5,130,338, which issued on July 14, 1992, refers to the use of sertraline to treat chemical dependencies, including dependencies on alcohol, tobacco and cocaine.

United States Patent 4,962,128, which issued on October 9, 1990, refers to the use of sertraline to treat disorders such as panic disorder, obsessive-compulsive disorder, generalized anxiety disorder, phobias, post traumatic stress disorder and avoidant personality disorder.

United States Patent 4,940,731, which issued on July 10, 1990, refers to the use of sertraline to treat premature ejaculation.

International Patent Application PCT/IB 95/00320, which designates the United States and was filed on May 4, 1995, refers to the use of sertraline to treat cancer patients.

United States Patent 5,114,976, which issued on May 19, 1992 discloses the use of Serotonin re-uptake blochers to treat patients suffering from stress

Examples of pharmaceutically acceptable salts of sertraline that can be used to treat post myocardial Infarction patients in accordance with the present invention are the acid addition salts of various mineral and organic acids such as hydrochloric, hydrobromic, hydroiodide, sulfuric, phosphoric, acetic, lactic, maleic, fumaric, citric, tartaric, succinic, and gluconic. Such salts may exist in one or more distinct crystalline forms or polymorphs, as well as in an amorphous state. Several crystalline polymorphs of the hydrochloride salt of sertraline are described in United States Patent 5,248,699, which issued on September 28, 1993.

Sertraline, its pharmaceutically acceptable salts and the different crystalline polymorphs of sertraline hydrochloride may be prepared as described in United States Patent 4,536,518, and particularly, in Example 2 of that patent, and in United States Patent 5,248,699.

Disclosed herein is a method of treating post myocardial infarction human patients comprising administering to such patients from about 12.5 mg/day to about 300 mg/day of sertraline or a pharmaceutically acceptable salt of sertraline.

"A method of treating", as used herein, refers either to a method of reducing the risk of death resulting from arrhythmia (sudden cardiac death) in post myocardial infarction human patients or to a method of improving the quality of life for such patients.

"Reducing the risk of death resulting from arrhythmia", as used herein, means effecting a clinically significant decrease in the probability or likelihood that a post myocardial infarction patient will experience death resulting from arrhythmia within a period of time following a myocardial Infarction, relative to what such probability or likelihood would be if such patient was not being treated with sertraline during such period. While the period of time that sertraline is administered to a post myocardial infarction patient to reduce the risk of death resulting from arrhythymia will be determined in each case by the prescribing physician, such period will generally begin after the occurrance of a myocardial infarction and end one to two years later.

"Death resulting from arrhythmia" refers to death which has as its immediate cause arrhythmia.

It is believed that sertraline will reduce the risk of death resulting from arrhythymia in post myocardial infarction patients regardless of whether they are suffering from clinical depression, generalized anxiety disorder, panic disorder or another anxiety disorder. "Anxiety disorder", as used herein, refers to any disorder classified as an anxiety disorder in the Diagnostic and Statistical Manual of Mental Disorders published by the American Psychiatric Association. One embodiment of this invention relates to a method of reducing the risk of death resulting from arrhythymia in a post myocardial infarction human patient who is not suffering from clinical depression, generalized anxiety disorder or another anxiety disorder, comprising administering to such patient sertraline, or a pharmaceutically acceptable salt of sertraline, in an amount from about 12.5 mg/day to about 300 mg/day, preferably from about 50 mg/day to about 200 mg/day.

The present inventor also believes that sertraline will be useful in improving the quality of life of post myocardial infarction patients. Quality of life can be determined according to any quality of life scale or standard that is recognized by those skilled in the art. Examples are the Katz Activities of Daily Living Scale (Katz et al., Int. J. Health Serv., 6, 493-507 (1976)) and the Sickness Impact Profile (Bergner et al., Med. Care, 14, 57-67 (1976)). The use of these and other parameters to evaluate the quality of life of patients in extremity sarcoma clinical trials is described by Sugarbaker et al., Surgery, 91, 17-23 (1982). Sertraline is expected to be useful in improving the quality of life of post myocardial infarction patients regardless of whether they are suffering from clinical depression, generalized human anxiety disorder, panic disorder or another anxiety disorder. One embodiment of this invention relates to improvement of the quality of life of a post myocardial infarction human patient who is not suffering from clinical depression, generalized anxiety disorder or another anxiety disorder, comprising administering. to such patient sertraline, or a pharmaceutically acceptable salt of sertraline, in an amount from about 12.5 mg/day to about 300 mg/day, preferably from about 50 mg/day to about 200 mg/day.

Sertraline, or a pharmaceutically acceptable salt thereof, when used to treat a post myocardial infarction patient may be administered either orally or parenterally. It is generally administered in dosages ranging from about 12.5 to about 300 mg per day, preferably from about 50 to about 200 mg per day, although variations will necessarily occur depending upon the condition of the subject being treated and the particular route of administration chosen. It may be administered either alone or in combination with pharmaceutically acceptable carriers by either of the above routes, and such administration can be carried out in both single and multiple dosages. More particularly, sertraline, or a pharmaceutically acceptable salt thereof, may be administered in a wide variety of different dosage forms, i.e., it may be combined with various pharmaceutically acceptable inert carriers in the form of tablets, capsules, lozenges, troches, hand candies, powders, sprays, aqueous suspensions, injectable solutions, elixirs, syrups, and the like. Such carriers include solid diluents or fillers, sterile aqueous media and various non-toxic organic solvents, etc. Moreover, such oral pharmaceutical formulations can be suitably sweetened and/or flavored by means of various agents of the type commonly employed for such purposes. In general, sertraline, or a pharmaceutically acceptable salt thereof, when used to treat a post myocardial infarction patient, is present in such dosage forms at concentration levels ranging from about 0.5% to about 90% by weight of the total composition, i.e., in amounts that are sufficient to provide the desired unit dosage.

For purposes of oral administration, tablets containing various excipients such as sodium citrate, calcium carbonate and calcium phosphate may be employed along with various disintegrants such as starch, preferably potato or tapioca starch, alginic acid and certain complex silicates, together with binding agents such as polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in soft and hard-filled gelatin capsules; preferred fillers would also include lactose or milk sugar as well as high molecular weight polyethylene glycols. When aqueous suspensions and/or elixirs are desired for oral administration, the sertraline, or pharmaceutically acceptable salt thereof, may be combined with various sweetening or flavoring agents, coloring matter or dyes and, if so desired, emulsifying and/or suspending agents, together with diluents such as water, ethanol, propylene glycol, glycerin and various like combinations thereof.

For purposes of parenteral administration, solutions of sertraline, or a pharmaceutically acceptable salt thereof, in sesame or peanut oil or in aqueous propylene glycol or N,N-dimethylformamide may be employed, as well as sterile aqueous solutions of the water-soluble, non-toxic mineral and organic acid addition salts previously enumerated. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal injection purposes. In this connection, the sterile aqueous media employed are all readily obtainable by standard techniques well-known to those skilled in the art.

A typical dry solid pharmaceutical composition is prepared by blending the following materials together in the proportions by weight specified below:
Cis-(1 S)-N-methyl-4-(3,4-dichlorophenyl)-1,2,3,4-tetrahydro-1-naphthalenamine hydrochloride: 50
Sodium citrate: 25
Alginic acid: 10
Polyvinylpyrrolidone: 10
Magnesium stearate: 5
After the dried composition is thoroughly blended, tablets are punched from the resulting mixture, each tablet being of such size that it contains 100 mg of sertraline hydrochloride. Other tablets are also prepared in a similar fashion containing 5, 10, 25, and 50 mg of sertraline hydrochloride respectively, by using the appropriate amount of the naphthalenamine salt in each case.

Another typical dry solid pharmaceutical composition is prepared by combining the following materials together in the proportions by weight indicated below:
Cis-(1S)-N-methyl-4-(3,4-dichlorophenyl)-1,2,3,4-tetrahydro-1-naphthalenamine hydrochloride: 50
Calcium carbonate: 20
Polyethylene glycol, average molecular weight, 4000: 30
The dried solid mixture so prepared is then thoroughly agitated so as to obtain a powdered product that is completely uniform in every respect. Soft elastic and hard-filled gelatin capsules containing this pharmaceutical composition are *then* prepared, employing a sufficient quantity of material in each instance so as to provide each capsule with 50 mg of the active ingredient.

## Claims

1. The use of sertraline or a pharmaceutically acceptable salt thereof, for the preparation of a medicament for reducing the risk of death resulting from arrhythmia in a post myocardial infarction human patient.

2. The use as claimed in claim 1 which comprises administering to said patient 12.5 to 300 mg/day of sertraline or a pharmaceutically acceptable salt thereof.

3. The use according to claim 2 wherein sertraline or a pharmaceutically acceptable salt thereof, is administered in an amount of 50 to 200 mg/day.

4. The use according to claim 1 wherein the post myocardial infarction human patient is not suffering from clinical depression, generalised anxiety disorder, panic disorder or another anxiety disorder.

## Revendications

1. Utilisation de la sertraline, ou d'un sel pharmaceutiquement acceptable de celle-ci, pour la préparation d'un médicament permettant la réduction du risque de mort résultant d'une arythmie chez un patient humain en état de post-infarctus du myocarde.

2. Utilisation selon la revendication 1, comprenant l'administration audit patient de 12,5 à 300 mg/jour de sertraline ou d'un sel pharmaceutiquement acceptable de celle-ci.

3. Utilisation selon la revendication 2, dans laquelle la sertraline ou un sel pharmaceutiquement acceptable de celle-ci est administré en une quantité de 50 à 200 mg/jour.

4. Utilisation selon la revendication 1, dans laquelle le patient humain en état de post-infarctus du myocarde ne souffre ni de dépression clinique, ni de trouble d'angoisse généralisée, ni de trouble panique, ni d'un autre trouble d'angoisse.

## Patentansprüche

1. Die Verwendung von Sertralin oder eines pharmazeutisch akzeptablen Salzes davon für die Herstellung eines Medikaments zum Reduzieren des Todesrisikos in Folge von Arrhythmie bei einem menschlichen Post-Myocard-Infarkt-Patienten.

2. Die Verwendung gemäß Anspruch 1, die das Verabreichen von 12,5 bis 300 mg/Tag Sertralin oder eines pharmazeutisch akzeptablen Salzes davon an den Patienten umfasst.

3. Die Verwendung gemäß Anspruch 2, worin Sertralin oder ein pharmazeutisch akzeptables Salz davon in einer Menge von 50 bis 200 mg/Tag verabreicht wird.

4. Die Verwendung gemäß Anspruch 1, worin der menschliche Post-Moycard-Infarkt-Patient nicht an klinischer Depression, generalisierter Angststörung, Panikstörung oder einer anderen Angststörung leidet.
